# EUROPÄISCHE PATENTANMELDUNG

(11) **EP 1 550 441 A1**
(43) Veröffentlichungstag der Anmeldung: **06.07.2005**
(21) Anmeldenummer: 05000014.0
(22) Anmeldetag: 03.01.2005
(51) Int. Cl.: A61K 31/192, A61K 31/167, A61K 45/06

(54) **Zusammensetzungen für eine synergistische topische Therapie neuromuskulärer Schmerzen**

(30) Priorität: 05.01.2004 DE 102004001093
(71) Anmelder: BIONICS PHARMA GMBH, 82031 Grünwald bei München (DE)
(72) Erfinder: Liedtke, Rainer K., c/o BIONICS PHARMA GMBH, 82031 Grünwald b. München (DE)
(74) Vertreter: Leson, Thomas Johannes Alois, Dipl.-Ing.

(57) **Zusammenfassung**

Die Erfindung beschreibt die Methode und Zusammensetzung einer synergistischen topischen Therapie der Symptomatik neuromuskulärer Schmerzen. Dabei wird eine für intakte Haut oder offene Haut geeignete topische pharmazeutische Formulierung eingesetzt, die, in geeigneter Dosis-Relation, mit einem Natrium-Kanal Blocker aus der Klasse der Lokalanästhetika vom Ester- oder Amid-Typ und einem Stoff aus der Klasse nichtsteroidaler anti-inflammatorischer Antiphlogistika beladen ist, und die diese Stoffe gezielt auf oder unter die Hautregion freisetzt. Durch die gleichzeitige Hemmung der initialen zellulär inflammatorischen Schmerzfaktoren als auch der hierzu reaktiven Transmission neuronaler Schmerzimpulse erreicht diese Therapie eine pharmakologisch effektivere Senkung neuromuskulärer Schmerzen.

## Beschreibung

Die Erfindung betrifft die Methode und Zusammensetzung einer synergistischen topischen Therapie der Symptomatik gelenknaher oder gelenkferner neuromuskulärer Schmerzen, insbesondere zur Anwendung mittels topischer pharmazeutischer Formulierungen über die Haut oder Hautschichten.

Neuromuskuläre Schmerzen sind emotional negative Wahrnehmungen, die bei lokalen Schädigungen im Gewebe des Bewegungs- und Stützapparates, oder bei dessen Funktionsstörungen, bestehen und die durch physikalische, chemische, biologische oder pyschosomatische Einflüsse entstanden sein können. Sie betreffen somit als Gewebe insbesondere die funktionell involvierten Nerven, Muskeln, Sehnen und Gelenke sowie die hiermit geweblich oder funktionell assoziierten Haut, Knochen- und Knorpelanteile des Skeletts. Solche Symptom-Komplexe werden unter anderem auch als Myalgien oder muskuloskeletäre Schmerzen bezeichnet. Im weiteren, sowie in dessen Kontext, sollen aber Schmerzen der vorgenannten Art hier stets unter dem funktionell übergeordneten Begriff neuromuskulärer Schmerzen gemeint und verstanden sein.

Es ist bekannt, dass Schmerzsymptomatiken wie sie beispielsweise schon bei einfachen Muskelverspannungen und -zerrungen, bei Rückenbeschwerden, stumpfen Traumen, Gelenks- und Faszien-Beschwerden, aber auch bei operativen Interventionen, auftreten, primär durch eine geweblich-lokale Kaskade biochemischer Reaktionen hervorgerufen werden, die letztlich in einer Irritation und Funktionsstörung peripherer sensorischer (afferenter) Nerven mündet.

Die Prevalenz und Inzidenz neuromuskulär schmerzhafter Störungen ist, infolge ihrer häufigen Alltagsursachen, in der Gesamtbevölkerung sehr hoch. Diese zudem oft bewegungseinschränkenden Störungen gehören daher zu den häufigsten Ursachen, die zu einer Minderung oder einem Ausfall an Arbeitsfähigkeit fuhren.

Pharmakologisch werden diese Schmerzsymptomatiken derzeit überwiegend mit systemisch wirkenden, oralen oder injektablen, Analgetika und Antiphlogistika angegangen. Als ein dabei derzeit pharmakologisches Hauptprinzip zur Schmerzbehandlung oder zur Wiederherstellung von Bewegungsfunktionen werden sogenannte nichtsteroidale Antiphlogistika eingesetzt, die in der medizinischpharmazeutischen Fachwelt international als sogenannte NSAIDs, non-steroidal antiinflammatory drugs, bezeichnet sind.Chemisch zur Gruppe der NSAIDs gehören Stoffe wie Acetylsalicylsäure, Diclofenac und Ibuprofen. NSAIDs finden sich in zahlreichen pharmazeutischen Applikationsformen, beispielsweise systemisch als Tabletten, Suppositorien oder Spritzen, wie auch in topischen Mitteln zur Anwendung über die Haut.

In diesem Zusammenhang sei zur Art der Verabreichung hier noch vorab der Begriff "topische Anwendung" als Gegensatz zur systemischen Anwendung von Arzneimitteln dargestellt. Mit einer topischen Anwendung ist nachfolgend gemeint die lokale Verabreichung von Arzneistoffen. Im Bereich einer Schmerztherapie ist dies daher deren Verabreichung direkt an oder in eine schmerzhafte Region. Dies kann beispielweise mit einer Salbe über einer schmerzhaften Hautstelle der Fall sein. Der Wirkort ist dabei weitgehend identisch mit dem Verabreichungsort. Dies steht im Gegensatz zu einer systemischen Verabreichung, bei der ein Arzneistoff, beispielsweise mit Gabe einer Tablette, nach der Aufnahme über Magen und Darm erst über die Blutbahn im gesamten Körper verteilt wird und hiernach und nur zu einem Bruchteil auch an den Ort der schmerzhaften Ursache gelangt. Dementsprechend sind bei systemischer Anwendung der Verabreichungsort und der Wirkort nicht identisch. Die hier eingesetzten Begriffe wie "topische Anwendungsform" oder "topische Zusammensetzung" meinen daher pharmazeutische Zubereitungen, die für den Zweck einer topischen Anwendung erstellt sind.

Den systemischen Therapien mit NSAIDs gemeinsam ist eine signifikante Rate an unerwünschten Wirkungen. Dies sind insbesondere Magenstörungen und Ulcera, sowie Störungen der Nierenfunktion und des Herz-Kreislaufs. Eine systemische Anwendung von NSAIDs bei neuromuskulären Schmerzen wird somit durch deren Spektrum an unerwünschten Wirkungen limitiert.

Bei den deutlich nebenwirkungsärmeren topisch dermalen (transcutanen) Anwendungen der NSAIDs gilt der therapeutische Nutzen aber als umstritten, da hier deren analgetische Wirkung oft deutlich geringer ausfällt. Die topische Wirkungsabnahme der NSAIDs wird dabei, nach derzeitigen Ansichten, oft auf die geringere Absorptionsrate über die Haut zurückgeführt, somit nur quantitativ als Dosis-Wirkungs-Problem, interpretiert. Solcher Annahme soll hier aber noch eine pharmakodynamische Ursache entgegengestellt werden. So decken die NSAIDs als Antiphlogistika innerhalb der Gesamtkasakade des zellulären Schmerzmechanismus nur einige Ausschnitte ab, die sich auch als schmerzdämpfend auswirken. Dies sind insbesondere jene, die mit biochemisch geweblichen Entzündungsvorgängen einhergehen. So üben NSAIDs primär nur eine Hemmung der Cyclooxygenasen 1 und 2 (COX-1/2) aus, worüber eine Bildung von Prostaglandinen vermindert wird. Infolgedessen werden sie auch als COX-Inhibitoren bezeichnet. Prostaglandine sind hormonähnliche Substanzen, die in chemisch verschiedenen Formen verbreitet im menschlichen Körper vorkommen. Im Rahmen ihres biologisch sehr breiten Wirkungsspektrums spielen sie insgesamt eine Rolle bei der Entstehung von Entzündungsprozessen und Fieber. So steigern sie im Rahmen von Entzündungen u.a. die Empfindlichkeit von Schmerzrezeptoren, beinflussen die Kontraktion glatter Muskulatur und verstärken die Freisetzung verschiedener Hormone.
Demgegenüber besitzen die NSAIDs aber keine direkten Wirkungen auf die, für eine Schmerzwahrnehmung sehr bedeutsamen, Folgeprozesse der neuronalen Weiterleitung der Schmerzinformation von dem Ort der peripheren Schmerzentstehung zum Zentralnervensystem.

Dieser Wirkungsmangel der NSAIDs vermag auch das Auftreten einer Rate geringerer Therapieeffekte oder von therapeutischen Non-Respondem bei topischer Anwendung von NSAIDs zu erklären. Dies insbesondere dann, wenn die entzündliche Komponente im Gewebe nur geringer ausgeprägt ist.

In der Schmerztherapie haben sich bei einigen Anwendungsgebieten auch lokale Injektionen mit Lokalanästhetika als klinisch erfolgreich erwiesen. Diesen lag als Basiskonzept die Ausschaltung von Nervenbahnen durch deren Betäubung zugrunde. Hierüber sollten dann unerwünschte Reflex-Reaktionen unterbunden werden. Injektionstechniken sind aber invasiv, selbst schmerzhaft und sie setzen anatomisch eine erfahrene ärztliche Handhabung voraus.
Als ein hierzu weiter differenziertes pharmakologisches Prinzip hat sich eine nicht-invasive Anwendung von Lokalanästhetika mit topischen Pflastersystemen bei neuromuskulären Schmerzen erwiesen. Diese neue Therapie wurde erst von uns sowie auch erst in den letzten Jahren erfunden und eingeführt. Dies erfolgte mit Patent US 5,776,952, für den Bereich einer topischen Anwendung von Lokalanästhetika bei Rückenschmerzen, sowie mit US 5,840,755 für eine topische Behandlung von Kopfschmerzen. Erst hierbei zeigte sich auch, daß Lokalanästhetika nicht nur invasiv als konventionelle Betäubungsmittel einsetzbar sind, sondern dass diese bei der Bekämpfung neuromuskulärer Schmerzen auch nichtinvasiv, als spezifisch neural wirkende Analgetika, ansehbar und einsetzbar sind. Die hierbei eingesetzten Stoffe, überwiegend Amid- und Ester-Lokalanästhetika, z.B. das Lidocain vom Amid-Typ, zeigen als pharmakologischen Wirkungsmechanismus eine Hemmung des schnellen Natrium-Ionen-mftux in Nervenfasern, d.h. sie wirken als sogenannte Natrium-Kanal Blocker. Über diesen spezifischen Effekt blockieren sie die Impulsleitung in Nervenfasern, was prinzipiell erst einmal alle regional vorhandenen Nervenfasern betrifft. Da jedoch die sensorischen schmerzleitenden Fasern anatomisch dünner sind als die motorischen Fasern (e.g. Strichartz, G. R (Edit.): Local Anesthetics, Handbook of Experimental Pharmacology, Vol 81. Springer, Berlin-New York 1987), lassen sich hieraus, über die verabreichte topische Dosis, auch unterschiedliche Wirkeffekte differenzieren.
Ein zusätzlicher und wesentlicher Grundaspekt für solch einen dosissparenden transcutanen therapeutischen Ansatz war es afferente Schmerzsignale als bioelektrisch irreguläre neuronale Informationsmuster zum Zentralnervensystem anzusehen, die schon über geringere Dosen an Lokalanästhetika wieder regularisiert bzw. normalisiert werden können. Auch diese Voraussagen und impliziten Annahmen u.a. von US 5,776,952, US 5,840,755 einer Beeinflussung ektopischer Impulse durch Lidocain wurden hiernach erst in den letzten Jahren experimentell weiter verifiziert (Khodorova A., Meissner K., Leeson S., Strichartz G.R.: Muscle Nerve. 24; 634 (2001), Persaud N., Strichartz G.R. Pain. 99, 333(2002).
Eine peripher topisch transcutane Schmerzsenkung erfordert daher geringere Dosen an Lokalanästhetika, als solche mit denen eine Anästhesie der Nervenfunktion, d.h. deren vollständige Inaktivierung, erzeugt wird. Daher unterscheidet sich auch eine transcutane Analgesie durch ihre Selektivität auf Schmerzrezeptoren von einer transcutanen Anästhesie, die alle lokalen Rezeptoren beeeinflusst, somit auch eine Inaktivierung motorischer Funktionen herbeiführt, was im Falle des therapeutischen Ziels einer reinen Analgesie unerwünscht ist.

Diese Effekte von Natrium-Kanal Blockern vom lokalanästhethischen Typus erstrecken sich aber ihrerseits spezifisch nur auf diesen Ausschnitt der neuronale Ebene. Anders als die NSAIDs, verfügen sie über keine pharmakologischen Effekte auf die geweblich biochemisch enzündlichen Vorgänge, noch besitzen sie antipyretische Wirkungen.

Dieser Wirkungsmangel vermag daher auch das Auftreten einer Rate von geringeren Therapieeffekten oder auch therapeutischen Non-Respondern bei der topischen Anwendung von Natrium-Kanal Blockern vom lokalanästhetischen Typus erklären. Dies insbesondere dann, wenn hier stärkere entzündliche Gewebskomponenten vorliegen.

Insgesamt ergibt sich somit, daß die NSAIDs einerseits und die Natrium-Kanal Blocker vom Typ der Lokalanästhetika andererseits, in ihrem schmerzsenkenden Wirkungsmechanismus zellulär jeweils nur sehr unterschiedliche Ausschnitte der peripher lokalen Schmerzkaskade abdecken:
Während die NSAIDs primär über die COX-Hemmung, damit nur indirekt, eine Wirkung auf initiale schmerzauslösende Prozesse geweblicher Enzündungsvorgänge spielen, insbesondere die hierbei involvierten algetischen Mediatoren und lokalen Ödembildungen, entfalten die Natrium-Kanal Blocker vom Typus der Lokalanästhetika hier keine direkten Wirkungen. Diese wiederum zeigen ihre für eine Schmerzsenkung relevanten spezifischen Effekte erst auf der Ebene der sich hiernach reaktiv ausbildenden neuronalen Prozesse der Schmerztransmission und zentralen Schmerzperzeption.

Aus einer synoptischen Sichtweise zu den zellulären Abläufen bei Schmerz und Entzündung lässt sich dann die Folgerung ableiten, dass sich bei gemeinsamer Verabreichung eines NSAID und eines Natrium-Kanal Blockers aus der Klasse der Lokalanästhetika insgesamt ein funktioneller pharmakologischer Synergismus bemerkbar machen sollte, dies obgleich diese Stoffe für sich jeweils mechanistisch sehr unterschiedlich und an verschiedenen Zellstrukturen wirken.

Therapeutisch sollte eine gemeinsame lokale Verabreichung dieser Stoffe daher zu einer gegenseitigen Verstärkung ihrer Wirkung auf den Schmerz im Sinne einer Addition oder Potenzierung führen.

Der Erfindung liegt die Aufgabe zugrunde, die Symptomatik gelenknaher oder gelenkferner neuromuskulärer Schmerzen mit einer synergistischen topischen Therapie über die Haut oder offene Hautschichten zu verbessern.

Diese Aufgabe wird dadurch gelöst, daß eine für intakte oder offene Hautschichten geeignete topische pharmazeutische Formulierung eingesetzt wird, welche eine therapeutisch geeignete Dosis eines Natrium-Kanal Blockers aus der Klasse der Lokalanästhetika vom Ester- oder Amid-Typ und eines Analgetikums aus der Klasse nichtsteroidaler Antiphlogistika enthält, wobei die Dosen dieser Stoffe in einer geeigneten Dosis-Relation vorliegen, und welche die enthaltenen Stoffe gezielt auf oder in die unter der pharmazeutischen Formulierung liegende Hautregion freisetzt.

Um die Anwendung der Therapie zu verbreitern, ist in einer weiteren Ausbildung der Erfindung die topisch pharmazeutische Formulierung eine Salbe, ein Gel, Puder, Emulsion, Suspension oder Lotion, eine wässrige oder alkoholische Lösung, ein Spray, oder ein transcutanes Pflastersystem, wobei die in der pharmazeutischen Formulierung eingesetzten Wirkstoffe gemeinsam homogen oder in unterschiedlichen chemischen Phasen oder in unterschiedlichen physikalischen Geometrien angeordnet vorliegen können.

Um eine geeignete Therapie zu spezifizieren sind in der topisch pharmazeutischen Formulierung als Wirkstoffe Lidocain, als Natrium-Kanal Blocker aus der Klasse der Lokalanästhetika vom Amid-Typ, und Diclofenac als Stoff aus der Klasse nichtsteroidaler Antiphlogistika, eingesetzt, wobei diese Stoffe jeweils in einer Konzentration von 0,5% - 40% vorliegen können.

Um die Wirksamkeit und Verträglichkeit der Therapie zu verbessern ist in der topisch pharmazeutischen Formulierung als weiterer Natrium-Kanal Blocker aus der Klasse der Lokalanästhetika vom Amid- oder Ester-Typ eingesetzt, Tetracain, Prilocain, Bupivacain, Mepivacain, Etidocain, Procain, Benzocain, Propoxycain, Hydroxyprocain, Chloroprocain, Ambucain, Metabutoxycain, Proparacain, Paraethoxycain, Butacain, Isobucain, Hexylcain, Piridocain, Piperocain, Cyclomethycain, Procainamid, Dibucain, Pyrrocain oder Tolycain, wobei der Stoff in einer Konzentration von 0,5% - 40% vorliegen kann.

Um die Wirksamkeit und Verträglichkeit der topischen Therapie zu verbessern ist in der topisch pharmazeutischen Formulierung als weiterer Stoff aus der Klasse der nichtsteroidalen Antiphlogistika eingesetzt, Indomethacin, Piroxicam, Ibuprofen, Ketoprofen, Naproxen, Tenoxicam, Etofenamat, Mefenamin, Flufenaminsäure, Felbinac, Salicylsäure, Acetylsalicylsäure, Methylsalicylat, Diethylaminsalicylat, oder Hydroxyethylsalicylat, wobei dieser Stoff in einer Konzentration von 0,5% - 40% vorliegen kann.

Um die medizinische Anwendung der Therapie zu verbreitern wird die topisch pharmazeutische Formulierung eingesetzt zur Behandlung geschlossener neuromuskulärer Schmerzen und Gelenkschmerzen, von Prellungen, Dehnungen und Zerrungen der Muskulatur, bei neuromuskulär bedingten Rückenschmerzen, rheumatischen Muskelbeschwerden, myofascialen Schmerzen und Tenditiniden, bei entzündlichen Gelenksreizungen, Gelenksdistorsionen und Gelenksstauchungen.

Um die medizinische Anwendung der Therapie zu verbreitern wird die topisch pharmazeutische Formulierung eingesetzt zur Behandlung von neuromuskulären Schmerzen auf Basis lokaler Nervenirritationen oder Nervenverletzungen, einschliesslich der Symptome von Neurodermatitis.

Um die medizinische Anwendung der Therapie zu verbreitern wird die topisch pharmazeutische Formulierung eingesetzt zur Behandlung neuromuskulärer Schmerzen bei offenen Wunden sowie bei post-incisionalen Wundschmerzen nach chirurgischen Operationen.

Um die medizinische Anwendung der Therapie zu verbreitern wird die topisch pharmazeutische Formulierung medizinisch im Humanbereich wie auch im Veterinärbereich eingesetzt

Eine direkte synergistische Therapie neuromuskulärer Schmerzen mittels topisch pharmazeutischer Formulierungen, die sowohl ein NSAID (COX-Inhibitor) und einen Natrium-Kanal Blocker aus der Klasse der Lokalanästhetika vom Ester- oder Amid-Typ enthalten, wurde bisher medizinisch weder beschrieben noch eingesetzt. Dies erscheint nicht verwunderlich, da die jeweiligen pharmakologischen Wirkungsprofile und Wirkungsmechanismen der beiden Stoffklassen, wie auch deren bisherige medizinischpraktische Anwendungen, weit auseinanderliegen.

Zudem wurde eine nicht-invasive Therapie mit Lokalanästhetika in topischen Pflastersystemen bei neuromuskulären Schmerzen auch erst von uns in den letzten Jahren erfunden und eingeführt. Nach den dortigen ersten therapeutischen Erfahrungen hiermit fiel dann auch auf, dass bei einem Vorliegen stärkerer entzündlicher Komponenten im Gewebe für die Natrium-Kanal Blocker auch eine verminderte therapeutische Ansprechrate vorzuliegen scheint, dies somit einer pharmakodynamischen Ergänzungswirkung bedarf.

Ohne synoptische Analyse der zellulär sequentiellen Abläufe im lokalen Schmerzgeschehen, einschliesslich der von entzündlichen Vorgängen, wird somit der sich ergänzende gemeinsame Nutzen dieser Stoffklassen für eine neuromuskuläre Schmerztherapie nicht erkennbar oder offensichtlich. Erst über diese Analyse zeigt sich auch, daß die neue topische Therapie eine synergistische Schmerzsenkung erzeugt, die dual verläuft, und dabei sowohl bei der Phase der Schmerzentstehung wie auch deren reaktiver Folge der Schmerztransmission ansetzt. Dabei gleichen die eingesetzten Stoffe dann den jeweiligen Effektmangel der anderen Stoffklasse aus.

Klinisch gesehen werden damit sowohl die schmerzinduzierenden ödematösentzündlichen Gewebseffekte als auch die den Schmerz aufrechterhaltenden Irritationen in der sensorisch neuronalen Leitung reguliert. Dies zusammen führt dann lokal zu einer tiefergehenden Senkung der Schmerzsymptomatik. Die Effektbreite einer Beeinflussung sowohl der entzündlichen als auch neuralgischen Komponente, ist somit mit keinem der Einzelstoffe für sich allein erzielbar.

Der Effekt ist auch nicht über eine systemische Verabreichungen der Kombination dieser Stoffe möglich. Der synergistische Effekt lässt sich nur dann therapeutisch ausreichend darstellen, wenn die Intervention der beiden Komponenten auch in geeigneter Dosierung direkt am Ort einer schmerzinduzierenden Läsion erfolgt, was bei einer systemischen Dosierung nicht genügend steuerbar ist. Bei einer systemischen Anwendung bestünde zudem ein hohes Risiko unspezifischer Schäden. Anders als bei der topisch sehr sicheren Anwendung wäre beispielsweise die systemische Verabreichung eines Lokalanästhetikums ein hohes toxisches Risiko, u.a. über deren cardiovaskuläre Effekte.

Funktional wirken die beiden Stoffe bezüglich einer Schmerzsenkung gleichsinnig. Da sie aber gewebstopographisch unterschiedliche Angriffsziele haben, eröffnet dies als weiteren Vorteil die Möglichkeit eines additiven oder überadditiven (potenzierenden) Effektes mit Dosisreduktion, somit ein hierdurch noch weiter verbessertes Sicherheitspotenzial.

Insgesamt ergibt sich somit als Vorteil der Erfindung eine verbesserte Senkung der Gesamtsymptomatik des peripher neuromuskulären Schmerzes, da diese hierbei erstmals auch eine Hemmung beider Schmerzkomponenten, der geweblichentzündlichen wie auch der neuralgischen, ermöglicht, die dort stets beide zu einem gewissen Anteil vorhanden sind. Diese Feststellung ergab sich auch schon bei den ersten einzelnen medizinischen Fallbeobachtungen, bei denen mit topischen Formulierungen, ähnlich dem nachfolgenden Beispiel 1, dann auch bei neuromukulären Schmerzbildem bessere Schmerzsenkungen erzeugt werden konnten, als dies mit handelsüblichen topischen Formulierungen der Fall war, die nur die Einzelstoffe enthielten, z.B bei einer Gel-Fomulierung mit 2% Diclofenac oder mit einem Pflaster mit 5% Lidocain.

Beispiel 1. Ein allgemeines Anschauungsbeispiel der Ausgestaltung einer topischen pharmazeutischen Formulierung, ohne es jedoch hierauf begrenzen zu wollen, kann eine dem pharmazeutischen Fachmann vertraute Gel-Formulierung sein, bei der in 100 Gramm dieses Gels dann einerseits als NSAID Komponente 2 Gramm Diclofenac-Natrium, sowie andererseits als Komponente an Natrium-Kanal Blocker 4 Gramm Lidocain, als Hydrochlorid, enthalten sind. In diesem Beispiel ist somit die topische w/w Dosis-Relation NSAID zu Natrium-Kanal Blocker auf 1:2 eingestellt. Von diesem Gel können jeweils ca. 3 Gramm bedarfsweise 2 bis 4 mal täglich auf das zu behandelnde schmerzhafte intakte Hautareal gegeben werden.

Beispiel 2. Als ein weiteres Beispiel der Ausgestaltung einer topischen pharmazeutischen Formulierung, ohne es jedoch hierauf begrenzen zu wollen, kann eine dem pharmazeutischen Fachmann vertraute Pflasterformulierung hergestellt werden.

Auf dessen der Haut zugewandten Seite ist ein hautadhesives Polymer als Beschichtung aufgebracht. Dieses Polymer dient dabei sowohl als Haftmittel auf der Haut wie auch Reservoir und Kontrollelement für die Freisetzung der Inhaltsstoffe. In 1 Gramm dieser adhesiven Beschichtung können, jeweils in homogener Verteilung, als NSAID Komponente 25 mg Diclofenac-Natrium sowie als Komponente an Natrium-Kanal Blocker 50 mg Lidocain-Hydrochlorid, enthalten sein. Die Konzentrationen in dem topischen Pflaster sind aus pharmakokinetischen Erfordernissen höher angesetzt als im Gel. Auch in diesem Beispiel ist daher die w/w Dosis-Relation von NSAID zu Natrium-Kanal Blocker auf 1:2 eingestellt. Ein Pflaster solcher Art kann für bis zu 24 Stunden auf eine schmerzhafte Stelle der intakten Haut geklebt werden.

Zahlreiche weitere pharmazeutische Ausgestaltungen sind möglich. Beispielsweise durch Änderungen der Dosis-Relationen der beiden Wirktoffe in einem homogenen Gemisch, durch pharmazeutische Formulierungen bei denen sich die beiden Stoffe in jeweils unterschiedlichen Phasen befinden, oder durch Pflaster bei denen die beiden Stoffe nur an geometrisch unterschiedlichen Stellen aufgetragen sind.
Die Erfindung beschreibt die Methode und Zusammensetzung einer synergistischen topischen Therapie der Symptomatik neuromuskulärer Schmerzen. Dabei wird eine für intakte Haut oder offene Haut geeignete topische pharmazeutische Formulierung eingesetzt, die, in geeigneter Dosis-Relation, mit einem Natrium-Kanal Blocker aus der Klasse der Lokalanästhetika vom Ester- oder Amid-Typ und einem Stoff aus der Klasse nichtsteroidaler anti-inflammatorischer Antiphlogistika beladen ist, und die diese Stoffe gezielt auf oder unter die Hautregion freisetzt. Durch die gleichzeitige Hemmung der initialen zellulär inflammatorischen Schmerzfaktoren als auch der hierzu reaktiven Transmission neuronaler Schmerzimpulse erreicht diese Therapie eine pharmakologisch effektivere Senkung neuromuskulärer Schmerzen.

## Patentansprüche

1. Methode und Zusammensetzung einer synergistischen topischen Therapie der Symptomatik gelenknaher oder gelenkfemer neuromuskulärer Schmerzen, **dadurch gekennzeichnet, daß** eine für intakte oder offene Hautschichten geeignete topische pharmazeutische Formulierung eingesetzt wird, welche eine therapeutisch geeignete Dosis eines Natrium-Kanal Blockers aus der Klasse der Lokalanästhetika vom Esteroder Amid-Typ und eines Analgetikums aus der Klasse nichtsteroidaler Antiphlogistika enthält, wobei die Dosen dieser Stoffe in einer geeigneten Dosis-Relation vorliegen, und welche die enthaltenen Stoffe gezielt auf oder in die unter der pharmazeutischen Formulierung liegende Hautregion freisetzt.

2. Methode und Zusammensetzung nach vorhergehendem Anspruch, **dadurch gekennzeichnet, daß** die topische pharmazeutische Formulierung ein Gel, eine Salbe, Puder, Emulsion, Suspension oder Lotion, eine wässrige oder alkolholische Lösung, ein Spray, oder ein transcutanes Pflastersystem ist, und wobei die in der eingesetzten pharmazeutischen Formulierung eingesetzten Wirkstoffe gemeinsam homogen oder in unterschiedlichen chemische Phasen oder unterschiedlichen physikalischen Geometrien angeordnet vorliegen können.

3. Methode und Zusammensetzung nach vorhergehenden Ansprüchen, **dadurch gekennzeichnet, daß** in der topisch pharmazeutischen Formulierung als Wirkstoffe Lidocain, als Natrium-Kanal Blocker aus der Klasse der Lokalanästhetika vom Amid-Typ, und Diclofenac, als Stoff aus der Klasse nichtsteroidaler Antiphlogistika, eingesetzt sind, wobei diese Stoffe jeweils in einer Konzentration von 0,5% - 40% vorliegen können.

4. Methode und Zusammensetzung nach vorhergehenden Ansprüchen, **dadurch gekennzeichnet, daß** in der topischen pharmazeutischen Formulierung als ein weiterer Natrium-Kanal Blocker aus der Klasse der Lokalanästhetika vom Amid- oder Ester-Typ eingesetzt ist, Tetracain, Prilocain, Bupivacain, Mepivacain, Etidocain, Procaine, Benzocain, Propoxycain, Hydroxyprocain, Chloroprocain, Ambucain, Metabutoxycain, Proparacain, Paraethoxycain, Butacain, Isobucain, Hexylcain, Piridocain, Piperocain, Cyclomethycain, Procainamid, Dibucain, Pyrrocain oder Tolycain, wobei der Stoff in einer Konzentration von 0,5% - 40% vorliegen kann.

5. Methode und Zusammensetzung nach vorhergehenden Ansprüchen, **dadurch gekennzeichnet, daß** in der topischen pharmazeutischen Formulierung als ein weiterer Stoff aus der Klasse der nichtsteroidalen Antiphlogistika eingesetzt ist, Indomethacin, Piroxicam, Ibuprofen, Ketoprofen, Naproxen, Tenoxicam, Etofenamat, Mefenamin, Flufenaminsäure, Felbinac, Salicylsäure, Acetylsalicylsäure, Methylsalicylat, Diethylaminsalicylat, oder Hydroxyethylsalicylat, wobei der Stoff in einer Konzentration von 0,5% - 40% vorliegen kann.

6. Methode und Zusammensetzung nach vorhergehenden Ansprüchen, **dadurch gekennzeichnet, daß** die topisch pharmazeutische Formulierung medizinisch zur Behandlung geschlossener neuromuskulärer Schmerzen und Gelenkschmerzen, bei Prellungen, Dehnungen und Zerrungen der Muskulatur, neuromuskulär bedingten Rückenschmerzen, rheumatischen Muskelbeschwerden, myofascialen Schmerzen und Tenditiniden, bei entzündlichen Gelenksreizungen, Gelenksdistorsionen und Gelenksverstauchungen eingesetzt wird.

7. Methode und Zusammensetzung nach vorhergehenden Ansprüchen, **dadurch gekennzeichnet, daß** die topisch pharmazeutische Formulierung medizinisch zur Behandlung neuromuskulärer Schmerzen auf Basis lokaler Nervenirritationen oder Nervenverletzungen einschliesslich der Symptome von Neurodermatitis, eingesetzt wird.

8. Methode und Zusammensetzung nach vorhergehenden Ansprüchen, **dadurch gekennzeichnet, daß** die topisch pharmazeutische Formulierung medizinisch zur Behandlung neuromuskulärer Schmerzen bei offenen Wunden und bei post-incisionalen Wundschmerzen nach chirurgischen Operationen eingesetzt wird.

9. Methode und Zusammensetzung nach vorhergehenden Ansprüchen, **dadurch gekennzeichnet, daß** die topisch pharmazeutische Formulierung medizinisch eingesetzt wird im Humanbereich wie auch im Veterinärbereich.
